(19) Europäisches Patentamt
European Patent Office
Office européen des brevets

(11) Publication number: 0 048 002
A2

(12) # EUROPEAN PATENT APPLICATION

(21) Application number: 81107199.2

(22) Date of filing: 11.09.81

(51) Int. Cl.³: C 07 D 475/08
//A61K31/505

(30) Priority: 12.09.80 DK 3904/80

(43) Date of publication of application:
24.03.82 Bulletin 82/12

(84) Designated Contracting States:
AT BE CH DE FR GB IT LI LU NL SE

(71) Applicant: Farmos Group Ltd.
Tengströminkatu 6 P.O. Box 425
SF-20101 Turku 10(FI)

(72) Inventor: Alster, Karol
Ellegaardspark 44
DK-3520 Farum(DK)

(72) Inventor: Jensen, Jens Karl
Berggreensgade 26
DK-2100 Copenhagen O(DK)

(72) Inventor: Svendsen, Bjorn
Stenholtsvej 20
DK-3480 Fredensborg(DK)

(74) Representative: Patentanwälte Grünecker,
Dr.Kinkeldey Dr.Stockmair, Dr.Schumann,Jakob,
Dr.Bezold Meister, Hilgers, Dr.Meyer-Plath
Maximilianstrasse 43
D-8000 München 22(DE)

(54) **Method for preparing N-(4-(((2,4-diamino-6-pteridinyl)-methyl)methylamino)benzoyl)glutamic acid.**

(57) The invention relates to a method for preparing N-[4-[[(2,4-diamino-6-pteridinyl)methyl]-methylamino]benzoyl]-glutamic acid (methotrexate), wherein diethyl N-[4-methylamino)benzoyl]glutamate of formula II

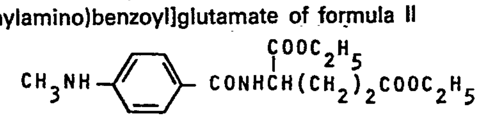

11

is alkylated with 2,4-diamino-6-chloromethylpteridine hydrochloride of formula I

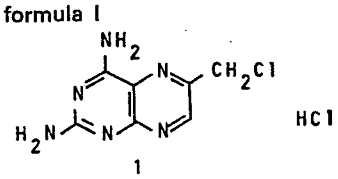

1

in a polar aprotic solvent in the presence of potassium iodide whereafter the resulting hydroiodide of diethyl N-[4-[[(2,4-diamino-6-pteridinyl)methyl]methylamino]benzoyl]-glutamate of formula III

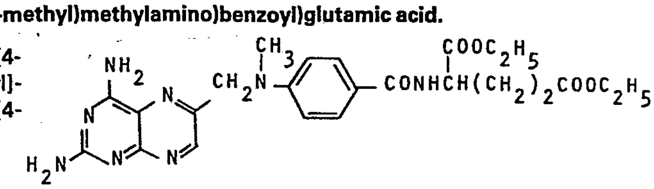

111

is hydrolyzed, either directly or after neutralization to III, to yield N-[4-[[(2,4-diamino-6-pteridinyl)methyl]methylamino]-benzoyl]glutamic acid (methotrexate) of formula V

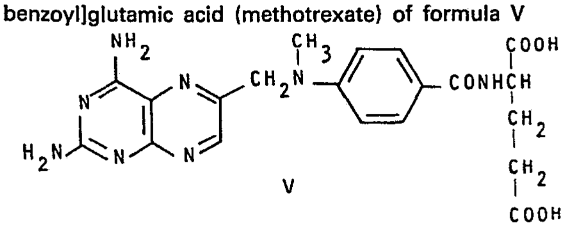

V

Methotrexate (N-[4-[[(2,4-diamino-6-pteridinyl)methyl]-methylamino]-benzoyl]glutamic acid) is a well known antineoplastic agent useful in the treatment of acute leukemia and other cancerous diseases.

EP 0 048 002 A2

The present invention relates to a method of preparing N-[4-[[(2,4-diamino-6-pteridinyl)methyl]methylamino]benzoyl]glutamic acid (methotrexate).

Methotrexate has been extensively used for over 30 years in cancer chemotherapy as an antineoplastic agent useful in the treatment of acute leukemia and other cancerous diseases. In the past, methotrexate has been accepted in a rather impure state and with unspecified optical rotation (cf. U.S.P. XIX 1973-74, p. 315 and B.P. 73, p. 300). The recent development of high-dose therapy prompted search for new methods of producing high purity methotrexate.

We have now developed an efficient and easy synthesis by which D-, L- or D,L-methotrexate of very high quality can be produced.

The method of the invention comprises alkylating diethyl N-[4-(methylamino)benzoyl]glutamate (II) with 2,4-diamino-6-chloromethylpteridine hydrochloride (I) in the presence of potassium iodide and hydrolyzing the resulting diethyl N-[4-[[(2,4-diamino-6-pteridinyl)methyl]methylamino]benzoyl]glutamate (III) to yield N-[4-[[(2,4-diamino-6-pteridinyl)methyl]methylamino]benzoyl]glutamic acid (methotrexate) of formula V

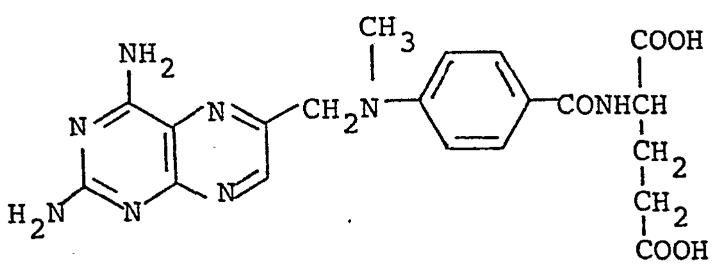

V

The hydrolysis is suitably performed by means of sodium hydroxide in aqueous ethanol. In the method of the invention, tedious purification procedures like column chromatography are not necessary; simple techniques like precipitation, centrifugation, and washing are sufficient to produce methotrexate of very high purity.

One major advantage of the process of the present invention is that no racemisation of the glutamic acid moiety takes place during the reactions. In this manner, the methotrexate obtained as the end product has the same optical configuration at the glutamic acid moiety as the starting material. That is, if the glutamic acid moiety in the compound II is in the L-form, L-methotrexate is obtained, which constitutes one of the preferred features of the present invention.

The starting material I, 2,4-diamino-6-chloromethylpteridine hydrochloride, is prepared in a very advantageous way by reacting 2,4-diamino-6-hydroxymethylpteridine (IV) with thionyl chloride at room temperature. When this reaction is performed in the absence of a solvent and in the absence of a catalyst, virtually pure 2,4-diamino-6-chloromethylpteridine hydrochloride (I) is obtained. The hydrochloride is much more soluble than the free base and is, hence, more suitable for the further synthesis.

One aspect of the invention is constituted by a method for preparing diethyl N-[4-[[(2,4-diamino-6-pteridinyl)methyl]methylamino]benzoyl]-glutamate (III) by alkylating diethyl N-[-4-(methylamino)benzoyl]-glutamate (II) with 2,4-diamino-6-chloromethylpteridine, hydrochloride (I) in the presence of potassium iodide. Compound III constitutes a valuable known intermediate in the production of methotrexate, and its further conversion into methotrexate by hydrolysis is, in principle, known per se.

The reaction between II and 2,4-diamino-6-chloromethylpteridine hydrochloride (I) is suitably performed in a polar aprotic solvent such as dimethylacetamide, dimethylformamide or, preferably, dimethylsulphoxide at 10 - 80$^{\circ}$C, preferably at ambient temperature, and the amount of potassium iodide is suitably 1 - 5 moles, calculated on I. The potassium iodide is preferably used in an amount

of 4 moles, calculated on I, and the reaction is preferably performed at room temperature, as these conditions ensure the highest yield and purity of the reaction product. The resulting hydroiodide of the compound III (which hydroiodide is novel <u>per se</u> and constitutes an aspect of the present invention) may be purified by recrystallization from methanol or neutralized with sodium hydroxide and the free base recrystallized. Both of these working up procedures lead to pure methotrexate after hydrolysis with sodium hydroxide in aqueous ethanol.

2,4-Diamino-6-hydroxymethylpteridine (IV) may be prepared from 5-acetoxymethyl-2-amino-3-cyanopyrazine according to the procedure of E.C. Taylor, J. Org. Chem. <u>40</u>, 2347 (1975). The complete reaction sequence starting from 5-acetoxymethyl-2-amino-3-cyanopyrazine is shown in the below reaction scheme:

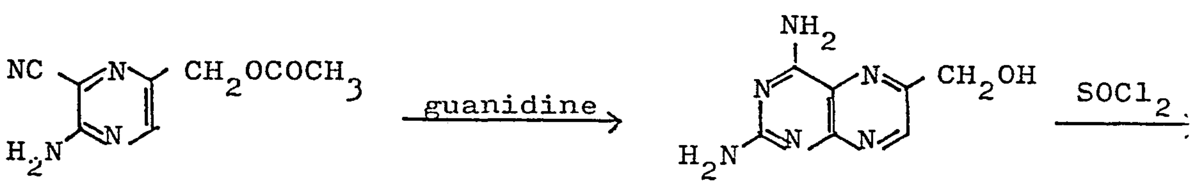


IV

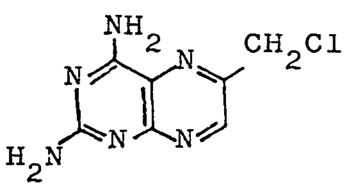

, HCl

I

I +

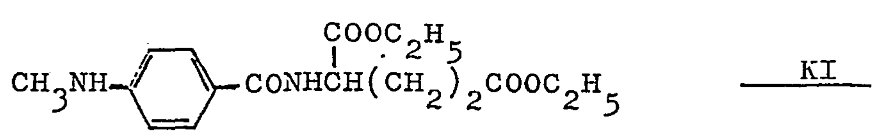


II

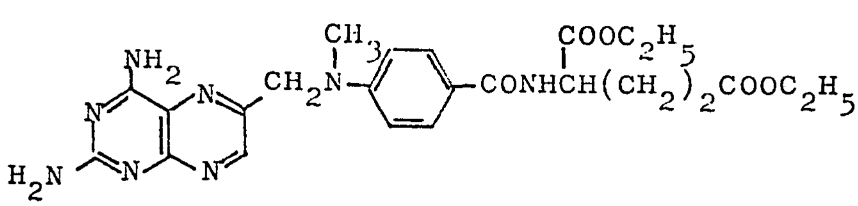

III

III $\xrightarrow{H_2O}$ Methotrexate (V)

The advantages of the principles of the present invention are, inter alia:

1) Methotrexate and intermediates are obtained in virtually pure form.
2) None or very simple purification procedures are required.
3) High yields are obtained in all steps of the synthesis.
4) No deterioration of stored intermediates is observed.
5) Only common reagents of technical grade need be used throughout the synthesis.
6) No racemisation takes place during the synthesis.

2,4-Diamino-6-chloromethylpteridine has previously been used in the synthesis of methotrexate (cf. British patent No. 1,414,752):

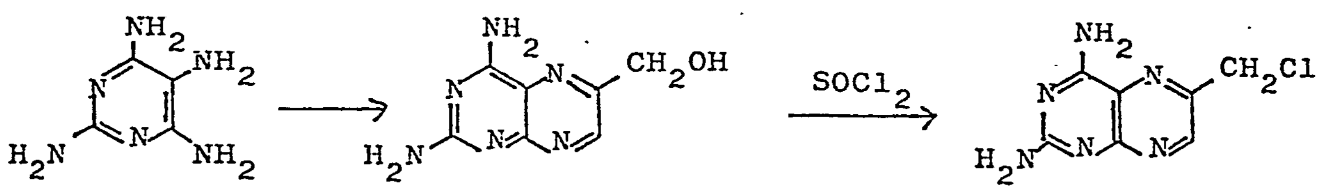


IV

Ia

Methotrexate (V)

According to the patent, the reaction IV ——> Ia was carried out in an inert medium like chloroform or methylene chloride under reflux in the presence of a basic catalyst, and Ia was obtained as the free base of unspecified purity. It appears from a study of the patent that the condensation of Ia with N-[4-(methylamino)benzoyl]glutamic acid gave methotrexate in a very low yield and of poor quality. This is in contrast to the present invention where the hydrochloride of Ia (compound I) was prepared in high yield and in high purity by a very simple process and was reacted, under special conditions, to form methotrexate of high purity.

The utilization of 2,4-diamino-6-halomethylpteridine in the production of methotrexate is also known from other publications and patents (J.R. Piper et al., J. Heterocycl. Chem. 11 (1974) 279, J.R. Piper

et al., J. Org. Chem. 42 (1977) 208, J.R. Piper et al., U.S. 4,079,056 and U.S. 4,077,957, J.A. Ellard, U.S. 4,080,325, E. Catalucci, Ger. Offen. 2,741,383). These authors utilize 2,4-diamino-6-bromomethylpteridine hydrobromide (Ib) as the alkylation agent under carefully selected reaction conditions. Three-step synthesis of Ib from 2,4,5,6-tetraaminopyrimidine is described in detail in the above references. The main disadvantage of this procedure is the formation of by-product pteridines. Careful monitoring of the reaction conditions is necessary to minimize the amount of contaminates.

Example 1.

2,4-Diamino-6-chloromethylpteridine hydrochloride (I).

2,4-Diamino-6-hydroxymethylpteridine (IV) (29.2 g, 0.151 mol) was added in one portion to thionyl chloride (290 ml) at room temperature. The reaction mixture was vigorously stirred for 60 minutes at room temperature, for additionally 90 minutes at 50°C, and again at room temperature overnight. Chloroform (500 ml) was added, and the resulting suspension was stirred under reflux for 2 hours. After cooling to 20°C, the solvent was removed by filtration. The solid was washed with chloroform and dried at 50°C. I (36 g, 96%) was hereby obtained as dark-yellow crystals, m.p. >300°C.

Calculated for $C_7H_8Cl_2N_6$ (247.2) + 4.6% $H_2O$ + 0.4% S:

| | | | | | | |
|---|---|---|---|---|---|---|
| | C 32.4 | H 3.6 | Cl 27.4 | N 32.4 | S 0.4 | $H_2O$ 4.6 |
| Found: | C 32.8 | H 3.8 | Cl 26.5 | N 31.6 | S 0.4 | $H_2O$ 4.6 (KF) |

This product was used in the next step of the synthesis without purification.

Example 2.

L-Methotrexate.

To the stirred mixture of I (8.8 g, 35.6 mmol) and II in the L-form (16.8 g, 49.9 mmol) in dimethylsulphoxide (47 ml), potassium iodide (23.6 g, 142 mmol) was added. A clear, dark solution occurred after 20 minutes. The reaction mixture was stirred at room temperature for 40 hours and then poured into a mixture of ethanol (50 ml) and water (160 ml). The dark-yellow precipitate that formed was washed three times with water and allowed to dry in the air. 15.7 g (77%) of III, hydroiodide in the L-form was hereby obtained as orange-yellow crystals. This product was recrystallized from methanol and saponified with sodium hydroxide in aqueous ethanol. The alkaline solution was treated with charcoal and filtered, and pH was adjusted to 4 with mineral acid. The fine yellow crystals that formed were filtered, washed with water and ethanol, and allowed to dry in the air. L-Methotrexate was hereby obtained in 87% yield.

Calculated for $C_{20}H_{22}N_8O_5$ + 12.4% $H_2O$:

| | | | | |
|---|---|---|---|---|
| | C 46.3 | H 5.7 | N 21.6 | $H_2O$ 12.4 |
| Found: | C 46.5 | H 5.7 | N 21.8 | $H_2O$ 12.4 (KF) |

Optical rotation $[\alpha]^D_{22}$ = +19.0 ± 0.5° (c = 1, 0.1N NaOH).

IR (KBr) and PMR spectra were identical with those of an authentic sample.

The purity of the product exceeded 98% (by quantitative HPLC).

Example 2a.

L-Methotrexate.

The crude, wet III, hydroiodide in the L-form prepared as described in Example 2 was suspended in a mixture of ethanol (155 ml) and water (40 ml). The pH of the resulting suspension was adjusted to 9 by addition of aqueous sodium hydroxide. The solution was treated with charcoal, filtered, and extracted with chloroform. The organic phase was separated and the solvent distilled off in vacuum. The residual solid was purified by recrystallization from n-pentanol. III in the L-form as yellow crystals was hereby obtained in 64% yield and a purity over 95% (by HPLC). This product was saponified as described in Example 2 to give L-methotrexate in 87% yield.

The compound was microanalytically pure within ± 0.3% abs.

Optical rotation $[\alpha]_{22}^{D} = +19.2 \pm 0.5°$ (c = 1, 0.1N NaOH).

The IR (KBr) and PMR spectra were identical with those of an authentic sample.

The purity of the product exceeded 98% (by quantitative HPLC).

Example 2b.

D-Methotrexate.

By the same procedure and under the same reaction conditions as described in Example 2a I was condensed with diethyl N-[4-(methylamino)benzoyl]-D-glutamate. The ester was saponified to give D-methotrexate, $[\alpha]_{25}^{D} = -18.8 \pm 0.3°$ (c = 2, 0.1N NaOH).

The compound was microanalytically pure within ± 0.3% abs.

The IR (KBr) and PMR spectra were identical with those of an authentic sample.

The purity of the product exceeded 98% (by quantitative HPLC).

Example 2c.

D,L-Methotrexate.

By the same procedure and under the same reaction conditions as described in Example 2a I was condensed with racemic diethyl N-[4-(methylamino)benzoyl]glutamate to give, after saponification, D,L-methotrexate, $[\alpha]_{25}^{D} = 0 \pm 0.8°$ (c = 1, 0.1N NaOH).

The compound was microanalytically pure within ± 0.3% abs.

The IR (KBr) and PMR spectra were identical with those of an authentic sample.

The purity of the product exceeded 98% (by quantitative HPLC).

Claims.

1. A method for preparing N-[4-[[(2,4-diamino-6-pteridinyl)methyl]-methylamino]benzoyl]glutamic acid (methotrexate), wherein diethyl N-[4-(methylamino)benzoyl]glutamate of formula II

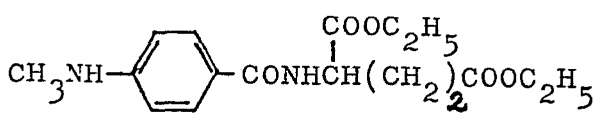,

II

is alkylated with 2,4-diamino-6-chloromethylpteridine hydrochloride of formula I

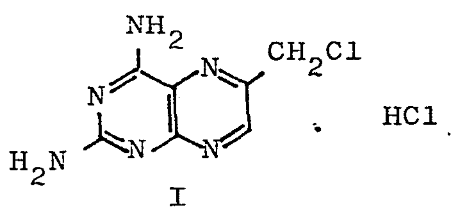

I

in a polar aprotic solvent in the presence of potassium iodide whereafter the resulting hydroiodide of diethyl N-[4-[[(2,4-diamino-6-pteridinyl)methyl]methylamino]benzoyl]glutamate of formula III

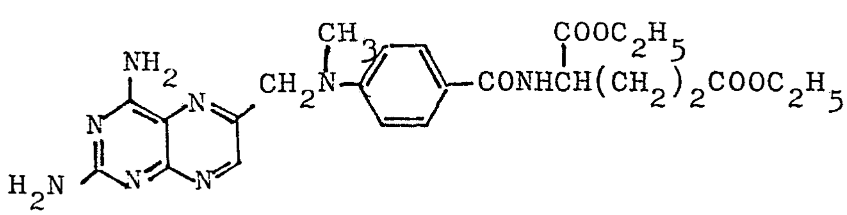

III

is hydrolyzed, either directly or after neutralization to III, to yield N-[4-[[(2,4-diamino-6-pteridinyl)methyl]methylamino]benzoyl]glutamic acid (methotrexate) of formula V

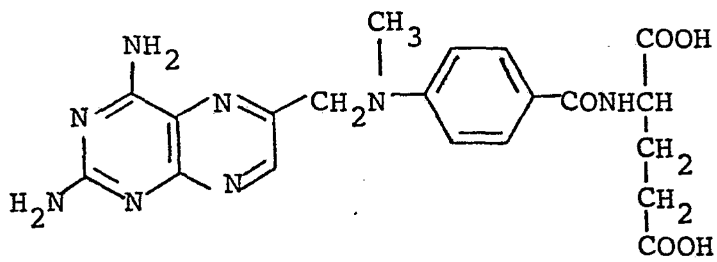

V

2. A method as claimed in claim 1, wherein the polar aprotic solvent is dimethyl sulphoxide.

3. A method as claimed in claim 1 or 2, wherein 1 - 5 mols of potassium iodide are used per mol of 2,4-diamino-6-chloromethylpteridine hydrochloride reacted.

4. A method as claimed in claim 3, wherein 4 mols of potassium iodide are used per mol of 2,4-diamino-6-chloromethylpteridine hydrochloride.

5. A method as claimed in any of claims 1 - 4, wherein the alkylation is carried out at a temperature from 10 to $80^{o}C$.

6. A method as claimed in claim 5, wherein the alkylation is carried out at ambient temperature.

7. A method as claimed in any of claims 1 - 6, wherein the 2,4-diamino-6-chloromethylpteridine hydrochloride of formula I is prepared by reacting 2,4-diamino-6-hydroxymethylpteridine of formula IV

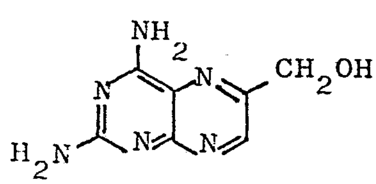

IV

with thionyl chloride at room temperature.

8. A method as claimed in claim 7, wherein the reaction is carried out by mixing 2,4-diamino-6-hydroxymethylpteridine with thionyl chloride in the absence of a solvent and in the absence of a catalyst.

9. A method for preparing diethyl N-[4-[[(2,4-diamino-6-pteridinyl)-methyl]methylamino]benzoyl]glutamate of formula III

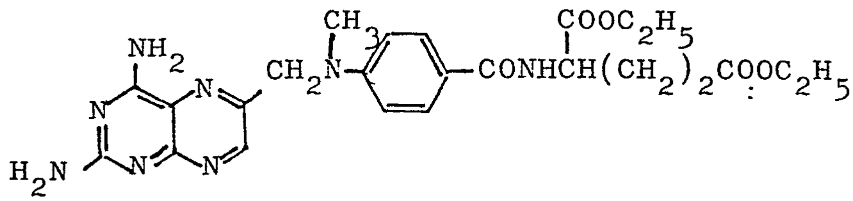

III

wherein diethyl N-[4-(methylamino)benzoyl]glutamate of formula II

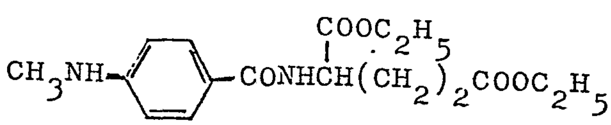

II

is alkylated with 2,4-diamino-6-chloromethylpteridine, hydrochloride of formula I

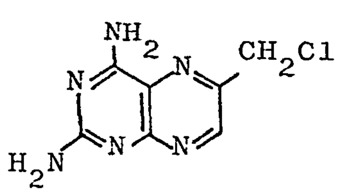

, HCl

I

in a polar aprotic solvent in the presence of potassium iodide, optionally followed by hydrolysis of the resulting hydroiodide of compound

III, either directly or after neutralization to III, to yield N-[4-[[(2,4-diamino-6-pteridinyl)methyl]methylamino]benzoyl]glutamic acid (methotrexate).

10. A method as claimed in claim 9, wherein the 2,4-diamino-6-chloromethylpteridine hydrochloride is prepared by reacting 2,4-diamino-6-hydroxymethylpteridine of formula IV

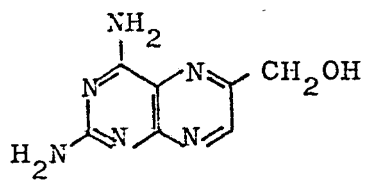

IV

with thionyl chloride at room temperature.

11. A method as claimed in claim 10, wherein the reaction is carried out by mixing 2,4-diamino-6-hydroxymethylpteridine with thionyl chloride in the absence of a solvent and in the absence of a catalyst.

12. Methotrexate whenever prepared by a process as claimed in any of claims 1 - 9.

13. D-Methotrexate whenever prepared by a process as claimed in any of claims 1 - 9.

14. L-Methotrexate whenever prepared by a process as claimed in any of claims 1 - 9.

15. D,L-Methotrexate whenever prepared by a process as claimed in any of claims 1 - 9.